# EUROPEAN PATENT APPLICATION

(11) **EP 1 568 366 A1**
(43) Date of publication of application: **31.08.2005**
(21) Application number: 04003694.9
(22) Date of filing: 19.02.2004
(51) Int. Cl.: A61K 31/4174, A61K 31/4164, A61P 31/10, A61K 47/02, A61K 47/18

(54) **Antifungal and/or antimycotic external preparation for nail comprising neticonazole**

(71) Applicant: Hisamitsu Medical Co., Ltd., Tokyo (JP)
(72) Inventor: Kawase, Ichiro, Narita-shi Chiba (JP); Ikeda, Yasuo, Narashino-shi Chiba (JP); Hara, Kousuke, Shiroi-shi Chiba (JP); Narui, Takashi, Sakura-shi Chiba (JP); Kaneko, Tetsuo, Narita-shi Chiba (JP)
(74) Representative: HOFFMANN EITLE

(57) **Abstract**

An antifungal and/or antimycotic external preparation for nail, which comprises neticonazole or a salt thereof and a basic substance.

## Description

### BACKGROUND OF THE INVENTION

### 1. Field of the Invention

The present invention relates to an antifungal and/or antimycotic external preparation for nail, which has good permeability of medicaments into nail and high therapeutic effect.

### 2. Brief Description of the Background Art

Among mycotic infectious diseases of the skin, onychomycosis (also called nail trichophytosis) causes cloudiness or yellowish white muddiness of nail. With the development of symptoms, thickening of a tip part of nail or the like deformation occurs. However, since there are no symptoms such as pain and itch, it advances and reaches deformation of the nail in many cases. Also, a case frequently occurs in patients of foot and hand mycotic infection, in which the symptoms temporarily end by the treatment with antifungal and/or antimycotic external preparations, but the diseases are generated again when the treatment is stopped. It is considered that the reason for this is due to discontinuation of the treatment under such a state that fungi existing in the deeper part of nail are not completely died out. Great concern has been directed toward the importance of the treatment of onychomycosis as a true meaning.

However, treatment of onychomycosis is markedly difficult, and its treatment with oral antifungal and/or antimycotic preparations is mainly carried out under the present situation, but the oral antifungal and/or antimycotic preparations and the like have a problem of frequently causing side effects such as liver dysfunction.

Thus, treatment of onychomycosis with external preparations has been attempted, but the efficacy is not sufficient.

### SUMMARY OF THE INVENTION

An object of the present invention is to provide an antifungal and/or antimycotic external preparation for nail, which has high permeability into nail and also has high efficacy.

This and other objects of the present invention have been accomplished by an antifungal and/or antimycotic external preparation for nail, which comprises the following components (a) and (b):
(a) neticonazole or a salt thereof;
(b) a basic substance.

### DETAILED DESCRIPTION OF THE INVENTION

The present inventors have carried out various examinations on antifungal and/or antimycotic preparations having high permeability into nail and external preparations containing the same and found that an antifungal and/or antimycotic external preparation for nail having high permeability into nail and also having high efficacy can be obtained when neticonazole or a salt thereof is combined with a basic substance. Thus, the present invention has been accomplished.

It is known that the neticonazole (component (a)) as the active ingredient of the antifungal and/or antimycotic external preparation for nail of the present invention (hereinafter referred to as "the preparation of the present invention"), (E)-1-[2-methylthio-1-[2-(pentyloxy)phenyl]ethenyl]-1H-imidazole in chemical name, is useful as an antifungal and/or antimycotic preparation as described in JP-B-6-45604. However, it is absolutely unknown that neticonazole has high nail permeability and is effective for nail trichophytosis. As the salt of neticonazole, an acid addition salt, particularly hydrochloride, is preferred.

The amount of the component (a) in the preparation of the present invention is preferably from 0.05 to 30% by weight, particularly from 0.5 to 15% by weight.

Examples of the basic substance as the component (b) include organic amines such as alkyl amines and mono-, di- and tri-alkanolamines; condensed products of fatty acid with alkanolamine; ammonia; and inorganic alkali such as alkali hydroxide and alkali carbonate. Among these, particularly, monoethanolamine, diethanolamine, triethanolamine, diisopropanolamine, diethanolamide oleate, ammonia, sodium hydroxide, potassium hydroxide and the like are preferred.

The amount of the component (b) in the preparation of the present invention is preferably from 0.01 to 10% by weight, particularly from 0.1 to 5% by weight.

According to the preparation of the present invention, it is preferred that the preparation or a 10-fold diluted aqueous solution of the preparation has a pH of 4 or higher. When the pH is lower, than 4, sufficient permeability of neticonazole into nail cannot be obtained and its efficacy cannot be obtained sufficiently. Also, the upper limit of the pH is not restricted, but is preferably 9 or less when irritability against the skin around nail is taken into consideration. The pH of the antifungal and/or antimycotic external preparation for nail of the present invention is most preferably from 5 to 8. The term "pH" as used herein means a pH of the antifungal and/or antimycotic external preparation for nail of the present invention in the case where it is an external preparation in which the pH can be directly measured (e.g., external preparation which contains water), or a pH of a 10-fold diluted aqueous solution (e.g., a pH of a supernatant obtained by adding 10 volumes of water, treating the mixture with ultrasonic wave for 1 hour and then centrifuging it) in the case where it is an external preparation in which the pH cannot be directly measured (e.g., an external preparation which does not contain water or contains less than 10% of water).

In addition, the antifungal and/or antimycotic external preparation for nail of the present invention can be further formulated with a surfactant, an oily component and the like.

Examples of the surfactant include polyoxyethylene alkyl ethers such as polyoxyethylene lauryl ether, polyoxyethylene cetyl ether, and polyoxyethylene oleyl ether; polyethylene glycol fatty acid esters such as polyethylene glycol monolaurate, polyethylene glycol monostearate, polyethylene glycol monooleate, and polyethylene glycol distearate; glycerol fatty acid esters such as glyceryl monostearate, glyceryl monooleate, and glyceryl dioleate; propylene glycol fatty acid esters such as propylene glycol oleate, propylene glycol monocaprylate, propylene glycol dicaprylate, and propylene glycol didecanoate; sorbitan fatty acid esters such as sorbitan monooleate; polyoxyethylene sorbitan fatty acid esters such as polyoxyethylene sorbitan monostearate and polyoxyethylene sorbitan monooleate; polyoxyethylene hydrogenated castor oil; sucrose fatty acid esters and the like. The surfactants may be used alone or as a mixture of two or more.

It is preferred that the blending amount of the surfactants is from 0.1 to 30% by weight, particularly from 0.5 to 20% by weight, further preferably from 1 to 15% by weight.

The oily component is not particularly limited, so long as it is a pharmaceutically acceptable compound. Examples include polyhydric alcohols, fatty acid esters, fatty acids, alcohols, medium chain fatty acid triglycerides and hydrocarbons. Specific examples include dibutyl adipate, diisopropyl adipate, diethyl sebacate, diisopropyl sebacate, isopropyl myristate, isopropyl palmitate, cetyl lactate, myristyl lactate and ethyl linoleate as the fatty acid esters; 1,3-butylene glycol, propylene glycol and dipropylene glycol as the polyhydric alcohols; lauric acid, oleic acid, linoleic acid and linolenic acid as the fatty acids; lauryl alcohol, oleyl alcohol, octyl dodecanol, benzyl alcohol and phenetyl alcohol as the alcohols; liquid paraffin and squalane as the hydrocarbons; medium chain fatty acid triglycerides and the like. The oily components may be used alone or as a mixture of two or more. It is preferred that the blending amount of the oily components is from 0.1 to 70% by weight, particularly from 1 to 65% by weight, based on the external preparation.

Also, in addition to the above components, the preparation of the present invention can contain an antioxidant, a stabilizing agent, a perfume, a coloring agent and the like.

The dosage form of the preparation of the present invention is preferably a dosage form of an adhesive preparation, a nail lacquer, a cataplasm, an ointment, a cream, a gel ointment, a solution or an aerosol. The amount contained of each of the above components in the present invention is the content in the total preparation in the case of ointments, creams, solutions and the like, but is the content in the base materials excluding the support in the case of adhesive preparations and cataplasms, or the content in the concentrated solution in the case of aerosols.

Materials for preparing such adhesive preparations are not particularly limited, so long as they are used in general adhesive preparations in addition to the above components. Examples include adhesive base materials such as natural rubber, polyisoprene rubber, polyisobutylene rubber, a styrene-isoprene-styrene block copolymer, a (meth)acrylic acid ester-(meth)acrylic acid copolymer, and a (meth)acrylic acid ester-vinyl compound copolymer; adhesiveness providing agents such as a rosin resin, a terpene resin and a petroleum resin (an aliphatic or alicyclic hydrocarbon resin); and softening agents such as a hydrocarbon compound, fatty acid, and a fatty acid ester; and the like.

As the backing to be used in this case, any material can be used without depending on its kind, so long as it is in a flexible sheet form in which the adhesive layer does not penetrate into the back side. Examples include woven cloth and nonwoven cloth; plastic films such as polyolefin film, polypropylene film, polyethylene film, polyvinyl alcohol film, vinyl chloride film, urethane alloy, urethane-vinyl chloride copolymer film, and ethylene-vinyl acetate film; foam films comprising a blend of acryl or polystyrene polybutadiene and polyisoprene; films prepared by depositing a metal on the above films; sheets prepared by laminating two or more of the respective films; and the like.

Materials for preparing nail lacquers are not particularly limited, so long as they are used in general nail lacquers in addition to the above components. Examples include coat forming agents such as a methacrylic acid alkyl ester copolymer, nitrocellulose, an alkyd resin, an acrylic acid-styrene copolymer, an ethylene-vinyl acetate copolymer, a polyester resin, a soluble nylon, cellulose acetate phthalate, hydroxypropylcellulose, hydroxypropylmethylcellulose, methylcellulose, ethylcellulose, polyvinyl acetal diethylaminoacetate, polyvinyl alcohol, polyvinylpyrrolidone, a methoxyethylene-maleic anhydride copolymer, and copolyvidone; solvents such as ethyl acetate, butyl acetate, acetone, methyl ethyl ketone, methyl isobutyl ketone, diisopropyl adipate, diisopropyl sebacate, diethyl sebacate, ethanol, isopropanol, xylene, toluene, acetone, and triacetin; and the like.

Materials for preparing cataplasms are not particularly limited, so long as they are used in general cataplasms in addition to the above components. Examples include adhesives such as polyacrylic acid, partially neutralized polyacrylate, sodium polyacrylate, starch grafted acrylate, carboxymethylcellulose sodium, a carboxyvinyl polymer, hydroxypropylcellulose, hydroxypropylmethylcellulose, methylcellulose, ethylcellulose, polyvinyl acetal diethylaminoacetate, polyvinyl alcohol, polyvinylpyrrolidone, and a methoxyethylene-maleic anhydride copolymer; moisturing agents such as concentrated glycerol, propylene glycol, and polyethylene glycol, and 1,3-butylene glycol; crosslinking agents such as an aluminum compound, a magnesium compound, and a calcium compound; crosslinking rate adjusting agents such as sodium edetate, sodium metaphosphate, lactic acid, citric acid, and tartaric acid; fillers such as kaolin, titanium oxide, and highly anhydrous silicic acid; and the like.

As the support to be used in this case, any material can be used without depending on its kind, so long as it is in a backing in which the adhesive layer does not penetrate into the backside. Examples include woven cloth, nonwoven cloth, *etc*.; plastic films such as polyolefin film, polypropylene film, polyethylene film, polyvinyl alcohol film, vinyl chloride film, urethane alloy, urethane-vinyl chloride copolymer film, and ethylene-vinyl acetate film; foam films comprising a blend of acryl or polystyrene polybutadiene and polyisoprene; supports prepared by pasting the above films on woven cloth or nonwoven cloth; backings prepared by laminating two or more of the respective films; and the like.

Materials for preparing ointments are not particularly limited, so long as they are used in general ointments in addition to the above components. Examples include mineral oils such as yellow vaseline, white vaseline, paraffin, liquid paraffin, Plastibase, Zelen 50W, and silicone; oils and fats such as olive oil, soybean oil, and sesame oil; fatty acid higher alcohols such as cetanol and stearyl alcohol; fatty acid esters such as yellow beeswax, white beeswax, isopropyl myristate, isopropyl palmitate, diethyl sebacate, diisopropyl adipate, and medium chain fatty acid triglyceride; and the like.

Materials for preparing creams are not particularly limited, so long as they are used in general creams in addition to the above components. Examples include hydrocarbons such as light liquid paraffin, squalane, white vaseline, microcrystalline wax, and ceresin; waxes such as beeswax, spermaceti, and carnauba wax; fatty acid esters such as diethyl sebacate, diisopropyl adipate, cetyl lactate, cetyl palmitate, and myristyl myristate; aliphatic alcohols such as stearyl alcohol, octyldodecanol, hexyldecyl alcohol, and cetanol; triglycerides such as natural fatty acid triglyceride and medium chain fatty acid triglyceride; fatty acids such as palmitic acid, stearic acid, and myristic acid; and the like.

Materials for preparing gel ointments are not particularly limited, so long as they are used in general gel ointments in addition to the above components. Examples include thickener polymers such as a carboxyvinyl polymer, polyacrylic acid, a sodium polyacrylate, crosslinked branched polyacrylic acid, a crosslinked branched sodium polyacrylate, hydroxyethylcellulose, hydroxypropylcellulose, hydroxypropylmethylcellulose, hydrophobic hydroxypropylmethylcellulose, methylcellulose, carboxymethylcellulose sodium, polyvinyl alcohol, polyvinyl pyrrolidone, polyethylene oxide, a methyl vinyl ether-maleic anhydride copolymer, polyacrylamide, alginic acid, sodium alginate, propylene glycol alginate, gelatin, gum arabic, tragacanth gum, locust bean gum, guar gum, tamarind gum, xanthan gum, geran gum, carrageenan, and agar; polyhydric alcohols such as 1,3-butylene glycol, propylene glycol, dipropylene glycol, polyethylene glycol, and glycerol; fatty acid esters such as dibutyl adipate, diisopropyl adipate, diethyl sebacate, diisopropyl sebacate, isopropyl myristate, isopropyl palmitate, cetyl lactate, myristyl lactate, ethyl linoleate, isopropyl linoleate, propylene glycol oleate, propylene glycol monocaprylate, propylene glycol dicaprylate, and propylene glycol didecanoate; surfactants such as polyoxyethylene lauryl ether, polyoxyethylene cetyl ether, polyoxyethylene oleyl ether, and lauric acid diethanol amide; fatty acids such as lactic acid, lauric acid, oleic acid, linoleic acid, and linolenic acid; aliphatic alcohols such as lauryl alcohol, oleyl alcohol, benzyl alcohol, and phenetyl alcohol; lower alcohols such as ethanol and isopropanol; hydrocarbons such as liquid paraffin and squalane; and the like.

Materials for preparing solutions are not particularly limited, so long as they are used in general solutions in addition to the above components. Examples include lower alcohols such as ethanol and isopropanol; fatty acid esters such as dibutyl adipate, diisopropyl adipate, diethyl sebacate, diisopropyl sebacate, isopropyl myristate, isopropyl palmitate, cetyl lactate, myristyl lactate, and ethyl linoleate; polyhydric alcohols such as 1,3-butylene glycol, propylene glycol, dipropylene glycol, polyethylene glycol, and glycerol; fatty acids such as lauric acid, oleic acid, linoleic acid, and linolenic acid; aliphatic alcohols such as lauryl alcohol, oleyl alcohol, benzyl alcohol, and phenetyl alcohol; hydrocarbons such as liquid paraffin and squalane; surfactants such as polyoxyethylene alkyl ether, polyethylene glycol fatty acid ester, glycerol fatty acid ester, propylene glycol fatty acid ester, sorbitan fatty acid ester, polyoxyethylene sorbitan fatty acid ester, polyoxyethylene hydrogenated castor oil, and sucrose fatty acid ester; and the like.

Materials for preparing aerosols are not particularly limited, so long as they are used in general aerosols in addition to the above components. Examples include lower alcohols such as ethanol and isopropanol; fatty acid esters such as dibutyl adipate, diisopropyl adipate, diethyl sebacate, diisopropyl sebacate, isopropyl myristate, isopropyl palmitate, cetyl lactate, myristyl lactate, and ethyl linoleate; polyhydric alcohols such as 1,3-butylene glycol, propylene glycol, dipropylene glycol, polyethylene glycol, and glycerol; fatty acids such as lauric acid, oleic acid, linoleic acid, and linolenic acid; aliphatic alcohols such as lauryl alcohol, oleyl alcohol, benzyl alcohol, and phenetyl alcohol; hydrocarbons such as liquid paraffin and squalane; surfactants such as polyoxyethylene alkyl ether, polyethylene glycol fatty acid ester, glycerol fatty acid ester, propylene glycol fatty acid ester, sorbitan fatty acid ester, polyoxyethylene sorbitan fatty acid ester, polyoxyethylene hydrogenated castor oil, and sucrose fatty acid ester; and the like. In addition, propellants such as liquefied gasses. e.g., liquefied petroleum gas, dimethyl ether; and compressed gasses, e.g., carbon dioxide, can also be used in the aerosols.

The thus obtained preparation of the present invention is used by adhering, applying or spraying it to affected parts of nail trichophytosis from a container generally used for respective dosage form.

Next, the present invention is described below in more detail with reference to examples, but the present invention is not limited thereto.

### Example 1

| Adhesive preparations: | |
|---|---|
| Neticonazole hydrochloride | 1.0 g |
| Styrene-isoprene-styrene block copolymer | 25.0 g |
| Liquid paraffin | 56.95 g |
| Diethyl sebacate | 5.0 g |
| Rosin ester derivative | 5.0 g |
| Sorbitan sesquioleate | 3.0 g |
| Sodium hydroxide | 0.12 g |
| Purified water | 2.88 g |
| Dibutylhydroxytoluene | 0.05 g |
| Total | 100 g |

Using a kneader as the blender, the styrene-isoprene-styrene block copolymer was mixed with the softening agent and rosin ester derivative under heating at a temperature of from 120 to 160°C, a solution prepared by dissolving neticonazole hydrochloride and sodium hydroxide in purified water and other components were added thereto, and then the mixture was directly spread on a sheet of polyester cloth. The obtained sheet was further covered with a polypropylene release film and then cut into pieces having a desired size to obtain adhesive preparations.

### Example 2

| Nail lacquers: | |
|---|---|
| Neticonazole hydrochloride | 5.0 g |
| Ethylcellulose | 15.0 g |
| Diethyl sebacate | 10.0 g |
| Polyoxyethylene(9)lauryl ether | 2.0 g |
| Diisopropanolamine | 0.4 g |
| Dibutylhydroxytoluene | 0.05 g |
| Acetone | 32.55 g |
| Adjust with anhydrous ethanol to | 100 g |

Neticonazole hydrochloride, diethyl sebacate, polyoxyethylene(9)lauryl ether, diisopropanolamine and dibutylhydroxytoluene were dissolved and mixed in a mixed solution of anhydrous ethanol and acetone, and then ethylcellulose was dissolved therein and the total amount was adjusted to 100 g with anhydrous ethanol. The thus prepared nail lacquer solution was put into glass containers equipped with a brush to obtain nail lacquers.

### Example 3

| Cataplasms: | |
|---|---|
| Neticonazole hydrochloride | 1.0 g |
| Diisopropyl adipate | 5.0 g |
| Sodium polyacrylate | 2.0 g |
| Polyacrylic acid | 2.0 g |
| Carboxymethylcellulose sodium | 3.0 g |
| Propylene glycol | 5.0 g |
| Concentrated glycerol | 20.0 g |
| D-Sorbitol solution (70%) | 35.0 g |
| Light silicic anhydride | 4.0 g |
| Aluminum potassium sulfate, dried | 0.3 g |
| Sodium edetate | 0.05 g |
| Sodium hydroxide | 0.12 g |
| Purified water | 22.53 g |
| Total | 100 g |

Using a mixer as the blender, sodium polyacrylate and polyacrylic acid were dispersed in a mixed solution of concentrated glycerol. Separately, a solution prepared by dissolving neticonazole hydrochloride and sodium hydroxide in a mixed solution of purified water and propylene glycol was, together with other components, added to and mixed with the above dispersion, and the mixture was homogenized to prepare an adhesive. Next, the obtained adhesive was directly spread on a backing of polyester nonwoven cloth, further covered with a polypropylene release film and then cut into pieces having a desired size to obtain cataplasms.

### Example 4

| Ointments: | |
|---|---|
| Neticonazole hydrochloride | 1.0 g |
| Diethyl sebacate | 5.0 g |
| Sorbitan sesquioleate | 3.0 g |
| Sodium hydroxide | 0.12 g |
| Purified water | 2.88 g |
| Dibutylhydroxytoluene | 0.05 g |
| Sodium edetate | 0.05 g |
| White vaseline | 86.9 g |
| Total | 100 g |

White vaseline was melted at a temperature of from 70 to 80°C, and dibutylhydroxytoluene was dissolved therein. Other components separately mixed at room temperature were added thereto, and the mixture was stirred at a temperature of from 60 to 70°C and then cooled as such to 45°C to obtain ointments.

### Example 5

| Creams: | |
|---|---|
| Neticonazole hydrochloride | 1.0 g |
| Diethyl sebacate | 12.0 g |
| Stearyl alcohol | 4.0 g |
| White vaseline | 5.0 g |
| Glycerol monostearate | 3.0 g |
| Polyoxyethylene(25)cetyl ether | 2.0 g |
| Methyl parahydroxybenzoate | 0.2 g |
| Butyl parahydroxybenzoate | 0.1 g |
| Sodium edetate | 0.02 g |
| Triethanolamine | 0.47 g |
| Adjust with purified water to | 100 g |

An oil phase was obtained by dissolving and mixing diethyl sebacate, stearyl alcohol, white vaseline, glycerol monostearate and polyoxyethylene(25)cetyl ether at a temperature of from 60 to 70°C. Next, a water phase was obtained by dissolving neticonazole hydrochloride and other components in purified water. The thus obtained water phase was added to the oil phase, and the mixture was emulsified at a temperature of from 70 to 75°C and then cooled to room temperature to obtain creams.

### Example 6

| Gel ointments: | |
|---|---|
| Neticonazole hydrochloride | 1.0 g |
| Carboxyvinylpolymer | 2.0 g |
| Ethanol | 25.0 g |
| Medium chain fatty acid triglyceride | 5.0 g |
| Polyoxyethylene(9)lauryl ether | 2.0 g |
| Triethanolamine | 3.0 g |
| Adjust with purified water to | 100 g |

While stirring a mixed solution of purified water and ethanol, neticonazole hydrochloride and other components were added thereto, and the mixture was stirred until the contents became uniform and then adjusted to 100 g with purified water, thereby obtaining gel ointments.

### Example 7

| Solutions: | |
|---|---|
| Neticonazole hydrochloride | 1.0 g |
| Octyl dodecanol | 30.0 g |
| Polyoxyethylene(10)monolaurate | 5.0 g |
| 1-Menthol | 0.5 g |
| Diisopropanolamine | 0.35 g |
| Ethanol | 45.0 g |
| Adjust with purified water to | 100 ml |

After dissolving neticonazole hydrochloride in ethanol, other components were added to and dissolved in the solution, and then the total volume was adjusted to 100 ml with purified water to obtain solutions.

### Example 8

| Aerosols: | |
|---|---|
| Concentrated solution: | |
| Neticonazole hydrochloride | 1.0 g |
| Medium chain fatty acid triglyceride | 30.0 g |
| Polyoxyethylene(9)lauryl ether | 5.0 g |
| 1-Menthol | 0.5 g |
| Diisopropanolamine | 0.4 g |
| Dibutylhydroxytoluene | 0.1 g |
| Adjust with anhydrous ethanol to | 100 ml |

| Filling: | |
|---|---|
| Concentrated solution | 18 ml |
| Liquefied petroleum gas | 42 ml |
| Total volume | 60 ml |

Neticonazole hydrochloride was added to and dissolved in a portion of anhydrous ethanol. Next, other components were added to and dissolved in the solution, and then the total volume was adjusted to 100 ml to prepare the concentrated solution of aerosols. The obtained concentrated solution of aerosols and liquefied petroleum gas were packed in an aluminum container that was then sealed with a bulb and equipped with an actuator and the like to thereby obtain aerosols.

### Comparative Example 1

Ointments were produced in the same manner as in Example 4, except that 0.12 g of sodium hydroxide in the prescription of Example 4 was excluded.

### Test Example 1

Using a pH meter, pH values of the antifungal and/or antimycotic external preparations for nail obtained in Examples 1 to 8 were measured. The pH values were directly measured in Examples 3, 5, 6 and 7, and in the case of Examples 1, 2, 4 and 8, each external preparation was diluted 10 times with water, treated with an ultrasonic wave for 1 hour and then centrifuged, and pH of the resulting supernatant was measured.

**Table 1**

| | Ex. 1 | Ex. 2 | Ex. 3 | Ex. 4 | Ex. 5 | Ex. 6 | Ex. 7 | Ex. 8 |
|---|---|---|---|---|---|---|---|---|
| pH | 6.3 | 5.4 | 6.2 | 6.5 | 6.4 | 6.0 | 6.1 | 5.5 |

### Test Example 2

### Testing method:

The dorsal surface of a human nail plate was polished with a sandpaper until the nail surface became dull, the thus nail was placed on absorbent cotton moistened with 1 ml of saline. After 30 minutes, about 5 mg of the preparation of Example 4 was applied to an area of 2 x 5 mm on the nail dorsal surface. The nail plate was removed 72 hours after application, the dorsal surface of nail plate was wiped out with a wiper and then wiped twice with a wiper dampened with methanol. Following the nail plate was wiped, the concentration of neticonazole in the nail was measured. Also, the amount of neticonazole permeated into the absorbent cotton was measured. The preparation of Comparative Example 1 was also tested in the same manner as the case of the preparation of Example 4.

### Measuring method:

The concentration of neticonazole in the human nail was measured by HPLC, after hydrolyzed with methanol and 5 mol/liter sodium hydroxide. Also, the amount of neticonazole in the absorbent cotton permeated through the human nail plate was measured by HPLC.

**Table 2**

| | Ex. 4 | Comp. Ex. 1 | Increasing ratio |
|---|---|---|---|
| Amount of neticonazole in the nail (ng) | 329.5 ± 70.3 | 53.0 ± 13.4 | 6.2 |
| Neticonazole permeated through the nail (ng/cm²) | 227.3 ± 454.5 | 23.3 ± 46.5 | 9.8 |

It can be understood from the results shown in Table 2 that the external preparation of the present invention has high permeability of neticonazole into nail and therefore is effective for nail trichophytosis which is difficult to be treated with conventional antifungal and/or antimycotic external preparations.

While the invention has been described in detail and with reference to specific embodiments thereof, it will be apparent to one skill in the art that various changes and modifications can be made therein without departing from the spirit and scope thereof. All references cited herein are incorporated in their entirety.

## Claims

1. An antifungal and/or antimycotic external preparation for nail, which comprises the following components (a) and (b):
(a) neticonazole or a salt thereof;
(b) a basic substance.

2. The antifungal and/or antimycotic external preparation for nail according to claim 1, wherein the preparation or a 10-fold diluted aqueous solution of the preparation has a pH of 4 or higher.

3. The antifungal and/or antimycotic external preparation for nail according to claim 1, which further comprises a surfactant.

4. The antifungal and/or antimycotic external preparation for nail according to claim 1, which further comprises an oily substance.

5. The antifungal and/or antimycotic external preparation for nail according to claim 1, which is in a dosage form of adhesive preparation, nail lacquer, cataplasm, ointment, cream, gel ointment, solution or aerosol.

## Amended claims

### Amended claims in accordance with Rule 86(2) EPC.

**1.** A use of
(a) neticonazole or a salt thereof; and
(b) a basic substance
for the manufacture of an external preparation for the treatment of nail mycosis.

**2.** The use according to Claim 1, wherein the nail mycosis is nail trichophytosis.

**3.** The use according to Claim 1 or 2, wherein the external preparation or a 10-fold diluted aqueous solution of the preparation has a pH of 4 or higher.

**4.** The use according to any of Claims 1 to 3, wherein the external preparation further comprises a surfactant.

**5.** The use according to any of Claims 1 to 4, wherein the external preparation further comprises an oily substance.

**6.** The use according to any of Claims 1 to 5, wherein the external preparation is in a dosage form of adhesive preparation, nail lacquer, cataplasm, ointment, cream, gel ointment, solution or aerosol.
